Europäisches Patentamt

European Patent Office

Office européen des brevets

(11) Veröffentlichungsnummer: **0 358 999**

**A1**

## (12) EUROPÄISCHE PATENTANMELDUNG

(21) Anmeldenummer: **89115632.5**

(22) Anmeldetag: **24.08.89**

(51) Int. Cl.5 **A61B 17/00**

(30) Priorität: **10.09.88 DE 3830909**

(43) Veröffentlichungstag der Anmeldung:
**21.03.90 Patentblatt 90/12**

(84) Benannte Vertragsstaaten:
**AT BE CH DE ES FR GB GR IT LI LU NL SE**

(71) Anmelder: **Astra Meditec AB
Box 14
S-431 21 Mölndal(SE)**

(72) Erfinder: **Borowka, Sonja, Dr. med.
St. Barbara Hospital Postfach 110115
D-4100 Duisburg 11(DE)**

(74) Vertreter: **Weber, Dieter, Dr. et al
Dr. Dieter Weber und Dipl.-Phys. Klaus
Seiffert Patentanwälte
Gustav-Freytag-Strasse 25 Postfach 6145
D-6200 Wiesbaden 1(DE)**

(54) **Krampfadersonde.**

(57) Die Erfindung betrifft eine Sonde zur Behandlung von Krampfadern, bestehend aus einem biegsamen Kunststoffrohr (1), welcher an seinem Vorderende ein Spitzenteil (2) aufweist, das durch Krampfadern hindurchführbar ist, und welcher mindestens die Länge der zu behandelnden Venen hat. Um das Einspritzen eines Sklerosiermittels an gewünschten Stellen in der Krampfadervene zu ermöglichen, wird erfindungsgemäß vorgeschlagen, daß die Sonde im Bereich des Spitzenteiles (2) eine seitliche Öffnung (3) aufweist und daß der innere Hohlraum (4) des Schlauches (1) von einem Ansatzstück (5) am hinteren Ende des Rohres (1) zu der seitlichen Öffnung (3) für das Einspritzen eines Sklerosiermittels eine Fließverbindung bildet.

Fig.1

# Krampfadersonde

Die vorliegende Erfindung betrifft eine Sonde zur Behandlung von Krampfadern, bestehend aus einem biegsamen Kunststoffrohr welches an seinem Vorderende ein Spitzenteil aufweist, das durch Krampfadern hindurchführbar ist, und welches mindestens die Länge der zu behandelnden Vene hat. Eine derartige Krampfadersonde ist beispielsweise bekannt aus der DE-PS 20 62 204. Die in dieser Patentschrift beschriebene sowie auch andere bekannte Krampfadersonden dienen im allgemeinen zum Entfernen (stripping) der kranken Venen. Dazu wird die Sonde mit ihrer Spitze an einem Ende in die Vene hinein und durch die gesamte Länge der zu entfernenden Vene hindurch und am anderen Ende durch eine chirurgische Öffnung wieder herausgeführt. Das Ende der Sonde verhakt sich an der Einführungsöffnung mit der Vene, so daß durch Ziehen am Spitzenteil die gesamte Vene herausgezogen wird. Dieser chirurgische Eingriff wird im allgemeinen unter Vollnarkose durchgeführt.

Zwar sind diese bekannten Venensonden teilweise auch schlauchförmig, jedoch ist der innere Rohrhohlraum im allgemeinen nicht für das Durchführen von Flüssigkeiten frei, sondern von einem Draht ausgefüllt, welcher zum einen Einfluß auf die Biegsamkeit bzw. Steifigkeit der Sonde hat, zum anderen aber auch dazu dient, die Zugkräfte beim Herausziehen der Vene aufzunehmen.

Zwar sind unter der Bezeichnung Katheter auch andere in Venen oder Arterien einführbare Sonden bekannt, jedoch bestehen diese entweder aus einem sehr weichen und biegsamen Kunststoffschlauch, welcher mit dem Blutstrom in die Vene oder Arterie mehr oder weniger eingeschwemmt wird, oder sie weisen einen Führungsdraht und eine mehr oder weniger scharfkantige Spitze auf, welche nur unter Röntgenkontrolle in das Blutgefäß eingeführt werden kann.

Ein Einführen des erstgenannten Kathetertyps in Krampfadern ist praktisch nicht möglich, da in diesen der Blutstrom mehr oder weniger zum Stillstand gekommen ist. Das Einführen des zweiten Kathetertyps unter Röntgenkontrolle ist sehr aufwendig und setzt Arzt und Patienten außerdem einer gewissen Strahlenbelastung aus. Darüber hinaus ist das Einführen herkömmlicher Katheter gerade in Krampfadern auch deshalb schwierig, weil die nicht mehr richtig funktionierenden Venenklappen und andere krankhafte Veränderungen dem Einführen eines herkömmlichen Katheters Widerstand entgegensetzen und die Gefahr von Verletzungen bzw. eines Durchstoßens der Venenwand nicht auszuschließen ist. Dagegen sind die bekannten Krampfadersonden, von welchen die Erfindung ausgeht, bei entsprechenem Geschick des Chirurgen ohne größere Mühe in die Venen einführbar.

Diese herkömmlichen Sonden haben jedoch den Nachteil, daß mit ihnen praktisch nur ein Herausreißen der Venen möglich ist, andere Behandlungsmethoden jedoch nicht zur Anwendung kommen können. Der vorliegenden Erfindung liegt daher die Aufgabe zugrunde, eine Krampfadersonde zu schaffen, mit welcher unter anderem auch ein Veröden (Sklerosieren) der Venen möglich ist.

Diese Aufgabe wird dadurch gelöst, daß die Sonde im Bereich des Spitzenteiles eine seitliche Öffnung aufweist und daß der innere Hohlraum des Rohres für das Einspritzen eines Sklerosiermittels eine Fließverbindung von einem Ansatzstück am hinteren Ende des Schlauches zur Öffnung im Spitzenteil bildet.

Gegenüber den herkömmlichen Venensonden füllt also ein Draht den Hohlraum des Kunststoffrohres nicht oder nicht vollständig aus, so daß eine Flüssigkeit durch die Sonde in die Vene eingespritzt werden kann, sofern das Rohr eine entsprechende Öffnung aufweist. Die Spitze des Rohres sollte jedoch keine Öffnung aufweisen, da sie zum einen des besseren Einführens wegen abgerundet sein sollte und da zum zweiten das Sklerosiermittel durch das vorhandene Rohrende nicht genügend ausgebracht werden kann. Dagegen ermöglicht die erfindungsgemäße, seitlich angebrachte Öffnung das Ausbringen des Sklerosiermittels genau an den Stellen, an denen es erwünscht ist. Dies sind insbesondere die Verbindungsstellen zum übrigen Venensystem.

Dabei erweist es sich als vorteilhaft, wenn die seitliche Öffnung sich im vorderen Drittel des Spitzenteiles befindet. Die zu behandelnden Venen können recht lang sein und das Sklerosiermittel muß unter Umständen an von der Einführungsöffnung sehr weit entfernten Stellen eingespritzt werden, so daß die seitliche Öffnung sich in vorteilhafter Weise möglichst weit vorn, das heißt im vorderen Drittel des Spitzenteiles befindet. Falls es gewünscht ist, kann jedoch die seitliche Öffnung weiter zurückversetzt sein und sich am rückwärtigen Ende des Spitzenteiles oder noch vor diesem befinden. Der Bereich des Spitzenteiles kann auch mehrere seitliche Öffnungen aufweisen.

Weiterhin hat es sich als vorteilhaft erwiesen, wenn das Spitzenteil spiralig gekrümmt ist. Eine solche Ausführungsform erleichtert das Einführen der Venensonde in die krankhaft veränderten Venen.

Schließlich kann es auch zweckmäßig und wünschenswert sein, wenn das Kunststoffrohr außer der Fließverbindung zwischen Ansatzstück und Spitzenöffnung auch noch eine Drahtverstärkung aufweist. Diese Drahtverstärkung kann im Rohrquerschnitt exzentrisch angebracht sein und kann unter anderem dazu dienen, der Sonde eine gewünschte Steifigkeit bzw. Biegsamkeit zu geben. Die Drahtverstärkung kann auch zentral im Rohrquerschnitt angebracht sein, wenn die Fließverbindung exzentrisch ist oder wenn der innere Hohlraum einen ausreichenden Querschnitt hat, um trotz Aufnahme des Drahtes auch als Fließverbindung dienen zu können.

Als Ansatzstück am hinteren Ende des Rohres kann ein herkömmliches Ansatzstück für Spritzen verwendet werden. Es kann jedoch auch der Rohrquerschnitt so gestaltet sein, daß eine Spritze oder ein anderes Zuführmittel für das Sklerosiermittel unmittelbar am hinteren Rohrende angesetzt werden kann. In diesem Fall wird das Ansatzstück sozusagen durch das abgeschnittene Rohrende selbst gebildet.

Weitere Vorteile und Merkmale der vorliegenden Erfindung werden deutlich anhand der folgenden Beschreibung einer bevorzugten Ausführungsform und der dazugehörigen Figur. Es zeigt:

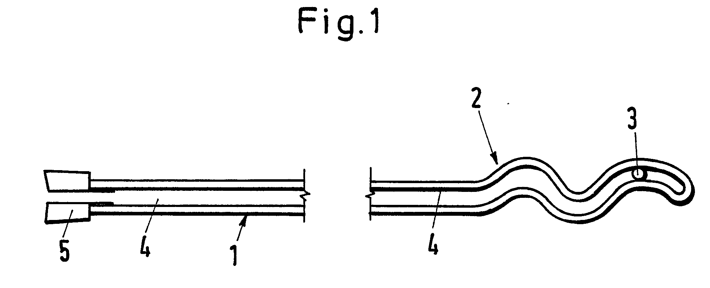

   Figur 1 schematisch den Aufbau einer Krampfadersonde.

Figur 1 zeigt, unterbrochen und im Längsschnitt, den Aufbau der neuen Krampfadersonde. Die Sonde besteht im wesentlichen aus einem Kunststoffrohr 1 mit einem inneren Hohlraum 4 und einem Spitzenteil 2. Das Spitzenteil 2 ist spiralig gewunden und an seinem Vorderende abgerundet. Am hinteren Ende weist die Sonde ein Ansatzstück 5 für eine Spritze auf. Dieses Ansatzstück 5 kann beispielsweise auch mit einem Ventil oder einer Durchstichmembran versehen sein. Ansonsten kann auch der Rohrquerschnitt von vorneherein so ausgebildet werden, daß sein hinteres Ende unmittelbar als Ansatzstück dient. Insbesondere kann eine solche Sonde serienmäßig in Maximallänge hergestellt und bei einer konkreten Verwendung gegebenenfalls um den gewünschten Betrag gekürzt werden, wobei das jeweils verbleibende hintere Ende jeweils als Ansatzstück dient. Unabhängig davon könnte ein Ansatzstück auch von dem Rohr lösbar sein und nach dem Kürzen wieder aufgesteckt werden.

Die seitliche Öffnung 3 befindet sich im vorderen Drittel des Spitzenteiles 2. Die spiralige Windung und die Abrundung des Vorderendes des Spitzenteiles 2 ermöglichen ein weitgehend problemloses Einführen der Sonde in die Vene. Vorzugsweise besteht das Kunststoffrohr aus einem zwar elastisch biegbaren aber doch ausreichend steifen Kunststoff.

Die Öffnung 3 ist deshalb seitlich am Spitzenteil 2 angebracht, weil auf diese Weise das Sklerosiermittel direkt in Bereiche gespritzt werden kann, in welchen Verbindungen zum übrigen Venensystem bestehen. Mit der neuen Sonde können Krampfadern auf ihrer ganzen Länge sklerosiert werden, indem während des Einführens oder des Herausziehens der Sonde aus der Vene das Sklerosiermittel über die ganze Länge der Vene verteilt durch die seitliche Öffnung eingespritz wird.

Sklerosiermittel bzw. Venenverödungsmittel, die durch die seitliche Öffnung eingespritzt werden können, sind seit längerem bekannt, wie zum Beispiel das Mittel Varigloban®.

Indem mit der neuen Sonde ein Veröden der Vene und insbesondere auch der Verbindungsbereiche zum benachbarten Venensystem möglich ist, kann das operative Entfernen der Krampfadern durch Herausziehen mit Hilfe der bekannten Krampfadersonden vermieden werden. Dies hat für den Patienten erhebliche Vorteile. Bei der Verwendung der neuen Sonde und der Anwendung des genannten Sklerosierverfahrens kommt man mit einer Lokalanästhesie aus, eine Vollnarkose ist nicht erforderlich.

Weiterhin kann der Eingriff ambulant vorgenommen werden, während bei dem herkömmlichen Herausziehen ("Stripping") der Krampfadern eine stationäre Behandlung erforderlich war. Außerdem ist das Ziehen der Vene wesentlich traumatischer und Hämatome sind ausgeprägter als bei der Verwendung der neuen Sonde.

Das Herausziehen der Venen führt überdies gelegentlich zu postoperativen Sensibilitätsstörungen, was bei der Anwendung der neuen Sonde und der Beschränkung auf Sklerosieren nicht zu befürchten ist.

Schließlich kann der Patient nach der Anwendung der neuen Sonde und dem Anlegen eines geeigneten Verbandes nach dem Eingriff uneingeschränkt laufen, während bei einer Behandlung nach dem herkömmlichen Verfahren mit den bekannten Sonden das Bein erst allmählich wieder belastbar ist.


**Ansprüche**

1. Sonde zur Behandlung von Krampfadern, bestehend aus einem biegsamen Kunststoffrohr (1), welches an seinem Vorderende ein Spitzenteil (2) aufweist, das durch Krampfadern hindurchführbar ist, und wobei das Rohr mindestens die Länge der zu behandelnden Vene hat, **dadurch gekennzeichnet,** daß die Sonde im Bereich des Spitzenteiles (2) mindestens eine seitliche Öffnung (3) aufweist und daß der innere Hohlraum (4) des Rohres (1) für das Einspritzen eines Sklerosiermittels eine Fließverbindung von einem Ansatzstück (5) am hinteren Ende des Rohres (1) zur Öffnung (3) am Spitzenteil (2) bildet.

2. Sonde nach Anspruch 1, **dadurch gekenn-**

zeichnet. daß die seitliche Öffnung (3) sich im vorderen Drittel des Spitzenteiles (2) befindet.

3. Sonde nach einem der Ansprüche 1 oder 2, **dadurch gekennzeichnet**, daß das Spitzenteil (2) spiralig gekrümmt ist.

4. Sonde nach einem der Ansprüche 1 bis 3, **dadurch gekennzeichnet**, daß das Kunststoffrohr (i) eine Drahtverstärkung aufweist.

# Fig.1

Europäisches Patentamt

**EUROPÄISCHER RECHERCHENBERICHT**

Nummer der Anmeldung

EP 89 11 5632

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-1 644 919 (HAYES) <br> * Seite 2, Zeilen 96-102; Figuren 2,3 * <br> --- | 1-4 | A 61 B 17/00 |
| Y,D | FR-A-2 075 300 (ASTRA-MEDITEC) <br> * Seite 2, Zeilen 27-32; Figur * <br> --- | 1-4 | |
| A | EP-A-0 170 969 (HAUER) <br> * Seite 5, Zeilen 10-18; Seite 10, <br> Zeilen 1-13; Figur 1 * <br> ----- | 1,4 | |
| | | | **RECHERCHIERTE SACHGEBIETE (Int. Cl.4)** |
| | | | A 61 B <br> A 61 M |

Der vorliegende Recherchenbericht wurde für alle Patentansprüche erstellt

| Recherchenort | Abschlußdatum der Recherche | Prüfer |
|---|---|---|
| DEN HAAG | 16-11-1989 | MOERS R.J. |

EPO FORM 1503 03.82 (P0403)